# EUROPÄISCHE PATENTANMELDUNG

(11) **EP 3 384 874 A1**
(43) Veröffentlichungstag der Anmeldung: **10.10.2018**
(21) Anmeldenummer: 17165560.8
(22) Anmeldetag: 07.04.2017
(51) Int. Cl.: A61F 2/24, A61B 17/04

(54) **ANULOPLASTIEVORRICHTUNG UND SYSTEM ZUR MINIMALINVASIVEN BEHANDLUNG EINER DYSFUNKTIONELLEN TRIKUSPIDALKLAPPE**

(71) Anmelder: Lauten, Alexander, 14532 Kleinmachnow (DE); Figulla, Hans Reiner, 07749 Jena (DE)
(72) Erfinder: FIGULLA, Hans Rainer, 07749 Jena (DE); DAMM, Christoph, 07743 Jena (DE); PESCHEL, Thomas, 07743 Jena (DE); KAMM, Andreas, 07743 Jena (DE)
(74) Vertreter: Trinks, Ole

(57) **Zusammenfassung**

Die Erfindung betrifft eine Anuloplastievorrichtung (100) zur minimalinvasiven Behandlung einer dysfunktionellen Herzklappe, insbesondere Trikuspidalklappe eines Patienten. Die Anuloplastievorrichtung (100) weist ein erstes und mindestens ein weiteres zweites Verankerungselement (2a, 2b) auf, welche jeweils ausgebildet sind, eine Verbindung mit dem Gewebe am Anulus (A) der zu behandelnden Herzklappe auszubilden. Des Weiteren weist die Anuloplastievorrichtung (100) ein Verbindungselement (4; 4a, 4b) auf, welches das erste Verankerungselement (2a) mit dem mindestens einen zweiten Verankerungselement (2b) verbindet, wobei die Länge des Verbindungselements (4; 4a, 4b) selektiv einstellbar ist.

## Beschreibung

Die vorliegende Erfindung betrifft eine Anuloplastievorrichtung sowie ein System zur minimalinvasiven Behandlung einer dysfunktionellen Trikuspidalklappe eines Patienten. Im Einzelnen betrifft die Erfindung Anuloplastievorrichtungen, die passiv dabei helfen, eine dysfunktionelle Herzklappe, insbesondere Trikuspidalklappe neu zu formen, um ihre Dichtheit zu verbessern.

Die Trikuspidalklappe ist die Herzklappe zwischen dem rechten Vorhof und der rechten Herzkammer. Sie ist eine Segelklappe und besteht beim Menschen normalerweise aus drei Segeln, welche mittels Sehnenfäden (chordae tendineae) mit den drei Papillarmuskeln der rechten Herzkammer verbunden sind.

Die Trikuspidalklappe wirkt als Rückschlagventil. Sie öffnet sich bei der Erschlaffung (Diastole) der rechten Herzkammer. Während der Systole schließt sich die Klappe durch den ansteigenden Druck und verhindert das Zurückfließen des Blutes in den rechten Vorhof. Das System aus Sehnenfäden und Papillarmuskeln verhindert, dass die Segel bei der Systole in den rechten Vorhof durchschlagen.

Ein mangelnder Verschluss der Klappe wird als Trikuspidal- oder Trikuspidalklappeninsuffizienz bezeichnet. Aus medizinischer Sicht stellt die operative Behandlung der Trikuspidalklappeninsuffizienz eine hohe Herausforderung dar. Dabei lässt die Dichtigkeit der Trikuspidalklappe nach, was dazu führen kann, dass aufgrund der Kontraktion des rechten Ventrikels Blutvolumen in den rechten Vorhof und die obere und/oder untere Hohlvene zurückgedrängt wird. Dies führt schlussendlich zu einer Volumenbelastung des rechten Herzens sowie zu einer Druckerhöhung im venösen Kreislaufsystem. In fortgeschrittenen Stadien kommt es zu einem Rechtsherzversagen mit Leberstauung und Ödembildung in peripheren Blutgefäßen kommen.

Je nach Schweregrad der Erkrankung ist es erforderlich, die natürliche Trikuspidalklappe vollständig durch eine künstliche Herzklappe oder durch einen Anuloplastiering oder dergleichen Vorrichtung zu ersetzen bzw. zu reparieren. Derartige chirurgische Eingriffe sind aufwendig und führen zu einer hohen Belastung des Patienten. Das bisherige Operationsverfahren wird am offenen Herzen durchgeführt, so dass der Patient während der Operation an eine Herz-Lungen-Maschine angeschlossen werden muss. Dies erhöht die Komplexität des Eingriffes und die Kreislaufbelastung für den Patienten. Entsprechend hoch ist die Morbidität und die Mortalität bei solchen Eingriffen.

Insbesondere im frühen Stadium einer Trikuspidalklappeninsuffizienz eingesetzte Verfahren bezieht die Verwendung von Anuloplastieringen ein, welche dazu dienen, die Trikuspidalklappenfunktion zu verbessern. Ein Anuloplastiering weist einen Durchmesser auf, der geringer ist als der Durchmesser des vergrößerten Klappenanulus der zu behandelnden Trikuspidalklappe. Der Ring wird im Klappenanulus platziert und das Gewebe des Anulus wird an den Ring genäht oder anderweitig gesichert. Dies bewirkt eine Reduktion des Anulusumfangs und einer Steigerung im Segelkoaptionsbereich und damit eine Verbesserung des Klappenschlusses und eine Verhinderung von Blutrückfluss.

Aufgrund der hohen Morbidität und Mortalität bei einem offenen chirurgischen Eingriffes an einer isolierten Trikuspidalklappe besteht Bedarf an einem minimalinvasiven Trikuspidalklappenreparatursystem, welches die Einschränkungen des Standes der Technik überwinden kann. Insbesondere besteht die Aufgabe der Erfindung darin, eine Anuloplastievorrichtung sowie ein System zur minimalinvasiven Behandlung einer dysfunktionellen Trikuspidalklappe anzugeben, die sich einfach minimalinvasiv und katheterbasierend chirurgisch implantieren lässt.

Erfindungsgemäß wird diese Aufgabe im Hinblick auf die Anuloplastievorrichtung durch den Gegenstand des Patentanspruches 1 und im Hinblick auf das System durch den Gegenstand des nebengeordneten Patentanspruches 10 gelöst, wobei vorteilhafte Weiterbildungen der erfindungsgemäßen Anuloplastievorrichtung bzw. des erfindungsgemäßen Systems in den jeweiligen abhängigen Patentansprüchen angegeben sind.

Demgemäß wird im Hinblick auf die Anuloplastievorrichtung zur minimalinvasiven Behandlung einer dysfunktionellen Trikuspidalklappe insbesondere vorgeschlagen, dass die Anuloplastievorrichtung ein erstes und mindestens ein weiteres, zweites Verankerungselement aufweist, wobei diese Verankerungselemente ausgebildet sind, eine Verbindung mit dem Anulus der zu behandelnden Trikuspidalklappe auszubilden.

Des Weiteren weist die erfindungsgemäße Anuloplastievorrichtung ein Verbindungselement auf, welches das erste Verankerungselement mit dem mindestens einen zweiten Verankerungselement verbindet, wobei die Länge des Verbindungselements selektiv einstellbar ist.

Vorzugsweise werden die Verankerungselemente derart am Anulus der zu behandelnden Trikuspidalklappe verankert, dass das die beiden Verankerungselemente verbindende Verbindungselement im Wesentlichen zentral über der Klappenöffnung platziert ist, um eine Segelkoaptation herzustellen und den Anulus zu verkleinern. Auf diese Weise kann minimalinvasiv die Trikuspidalklappe behandelt werden, ohne dass hierzu ein chirurgischer Eingriff mit einem Alfiere-Stitch oder eine Anuloplastie notwendig ist.

Eine Beschränkung dieser herkömmlicher Verfahren liegt insbesondere darin, dass diese typischerweise das Öffnen des Herzens erfordern, um einen direkten Zugang zu der zu behandelnden Trikuspidalklappe und zum Klappenanulus zu erhalten. Dies erfordert im Allgemeinen die Verwendung eines kardiopulmonalen Bypasses, was dem chirurgischen Verfahren eine zusätzliche Morbidität und Mortalität hinzufügt.

Zusätzlich ist es schwierig, bei den herkömmlichen operativen Verfahren am stillgelegten Herzen die Effektivität der Reparatur vor Abschluss der Operation zu beurteilen.

Indem erfindungsgemäß minimalinvasiv die Verankerungselemente am Anulus der zu behandelnden Trikuspidalklappe verankert werden und anschließend - wiederum minimalinvasiv - die Verankerungselemente miteinander über ein bezüglich seiner Länge einstellbaren Verbindungselements verbunden werden, kann wirksam der Querschnittsbereich des Klappenanulus reduziert und somit die Klappendysfunktion behandelt werden, ohne dass hierfür eine Operation am offenen Herzen erforderlich ist.

Insbesondere ist bevorzugt vorgesehen, dass sämtliche Komponenten der Anuloplastievorrichtung zur minimalinvasiven Behandlung im Allgemeinen transluminal, insbesondere transfemoral oder transjugulär, implantierbar sind. Dies gewährleistet einen minimalinvasiven Eingriff und reduziert die Belastung des Patienten während der Operation. So können die Verankerungselemente insbesondere einzeln über einen Katheter an den Behandlungsort geführt werden, wobei der Katheter vorzugsweise über die Femoralvene und die untere Hohlvene zum rechten Herzvorhof geschoben wird.

Alternativ kann ein Zugang über die Halsvene (vena jugularis) gewählt werden. Dabei werden die Verankerungselemente - separat, d.h. getrennt voneinander oder gleichzeitig - über die obere Hohlvene zum rechten Herzvorhof geschoben. In beiden Fällen werden die Verankerungselemente vorzugsweise über ein kathetergeführtes Werkzeug in das Gewebe am Anulus der zu behandelnden Trikuspidalklappe eingeschraubt und somit entsprechend verankert.

Das Verbindungselement, welches die Verankerungselemente miteinander verbindet, ist vorzugsweise biegsam in der Längsrichtung jedoch nicht oder zumindest im Wesentlichen nicht elastisch ausgeführt. Insbesondere bietet es sich hierbei an, ein Verbindungselement in Gestalt eines Fadens, beispielsweise Dacronfadens, oder eines mehrere miteinander verbundene oder verdrehte Fasern aufweisenden Fadens einzusetzen.

Um das Verbindungselement möglichst einfach mit den miteinander zu verbindenden Verankerungselementen zu verbinden, ist insbesondere vorgesehen, dass das Verbindungselement mehrteilig ausgeführt ist und einen ersten Bereich aufweist, welcher mit dem ersten Verankerungselement fest verbunden ist, und mindestens einen weiteren, zweiten Bereich aufweist, welcher mit dem mindestens einen zweiten Verankerungselement fest verbunden ist. Bei dieser Ausführungsform der erfindungsgemäßen Anuloplastievorrichtung kommt ferner ein vorzugsweise über einen Katheter minimalinvasiv einführbares Befestigungselement zum Einsatz, um den ersten Bereich des Verbindungselements mit dem mindestens einen zweiten Bereich des Verbindungselements zu verbinden.

Das Befestigungselement ist vorzugsweise derart ausgebildet, dass dieses den ersten Bereich des Verbindungselements mit dem mindestens einen zweiten Bereich des Verbindungselements derart verbindet, dass die Länge des Verbindungselements insgesamt selektiv einstellbar ist. Die Länge des Verbindungselements ist insbesondere so zu wählen, dass im implantierten Zustand der Anuloplastievorrichtung eine auf den Anulus der zu behandelnden Trikuspidalklappe einwirkende Kraft erzeugt wird, wodurch die Klappenfunktion der zu behandelnden Trikuspidalklappe verändert und insbesondere optimiert wird.

In vorteilhaften Ausführungsformen der vorliegenden Erfindung ist das Befestigungselement ausgebildet, den ersten Bereich des Verbindungselements mit dem mindestens einen zweiten Bereich des Verbindungselements kraftschlüssig, insbesondere klemmend, miteinander zu verbinden.

Die Erfindung betrifft ferner ein System zur minimalinvasiven Behandlung einer dysfunktionellen Trikuspidalklappe eines Patienten, wobei das System zusätzlich zu der vorstehend beschriebenen Anuloplastievorrichtung noch einen Einführmechanismus zum minimalen Einführen und Implantieren der einzelnen Komponenten der Anuloplastievorrichtung in dem zu behandelnden Herzen des Patienten aufweist. Insbesondere ist in diesem Zusammenhang vorgesehen, dass der Einführmechanismus ein kathetergeführtes Werkzeug aufweist zum Ausbilden der formschlüssigen Verbindung zwischen dem ersten und/oder mindestens einen zweiten Verankerungselement und dem Gewebe am Anulus der zu behandelnden Trikuspidalklappe.

Gemäß Ausführungsformen des erfindungsgemäßen Systems weist das kathetergeführte Werkzeug einen hohlkörperförmigen Bereich mit einer Wendelnut auf, wobei die Wendelnut derart an das am Anulus der zu behandelnden Trikuspidalklappe zu verankernden Verankerungselement angepasst ist, dass durch eine Rotation des hohlkörperförmigen Bereiches des kathetergeführten Werkzeuges um seine Längsachse das Verankerungselement zumindest bereichsweise in das Gewebe am Anulus der zu behandelnden Trikuspidalklappe einführbar ist.

Alternativ hierzu ist es aber auch denkbar, wenn das kathetergeführte Werkzeug einen hohlkörperförmigen Bereich mit einer Längsnut aufweist, wobei die Längsnut derart an das am Anulus der zu behandelnden Trikuspidalklappe zu verankernde Verankerungselement angepasst ist, dass durch eine Rotation des hohlkörperförmigen Bereiches des kathetergeführten Werkzeuges um seine Längsachse das Verankerungselement zumindest bereichsweise in das Gewebe am Anulus der zu behandelnden Trikuspidalklappe einführbar ist.

Die Erfindung wird im Folgenden anhand von Ausführungsbeispielen unter Bezugnahme auf die beigefügten, schematischen Zeichnungen näher erläutert.

Es zeigen:
- FIG. 1: schematisch eine exemplarische Ausführungsform eines Verankerungselements mit einem mit dem Verankerungselement verbundenen Verbindungselement in Gestalt eines Fadens;
- FIG. 2: bereichsweise eine exemplarische Ausführungsform des distalen Endbereiches eines kathetergeführten Werkzeuges zum Ausbilden einer formschlüssigen Verbindung zwischen einem Verankerungselement und dem Gewebe am Anulus einer zu behandelnden Trikuspidalklappe;
- FIG. 3: schematisch die exemplarische Ausführungsform des kathetergeführten Werkzeuges gemäß FIG. 2 mit einem im distalen Endbereich des Werkzeuges zumindest bereichsweise aufgenommenen Verankerungselement;
- FIG. 4: schematisch den Vorgang zum Implantieren der Verankerungselemente mit Hilfe des kathetergeführten Werkzeuges gemäß FIG. 2 am Anulus einer zu behandelnden Trikuspidalklappe;
- FIG. 5: bereichsweise eine weitere exemplarische Ausführungsform des distalen Endbereiches eines kathetergeführten Werkzeuges zum Ausbilden einer formschlüssigen Verbindung zwischen einem Verankerungselement und dem Gewebe am Anulus einer zu behandelnden Trikuspidalklappe;
- FIG. 6: schematisch die exemplarische Ausführungsform des kathetergeführten Werkzeuges gemäß FIG. 5 mit einem im distalen Endbereich des Werkzeuges zumindest bereichsweise aufgenommenen Verankerungselement;
- FIG. 7: schematisch den Vorgang zum Implantieren der Verankerungselemente mit Hilfe des kathetergeführten Werkzeuges gemäß FIG. 5 am Anulus einer zu behandelnden Trikuspidalklappe; und
- FIG. 8a-g: schematisch den Vorgang zum Verbinden von zwei am Anulus einer zu behandelnden Trikuspidalklappe implantierten Verankerungselementen.

Die Erfindung betrifft eine Anuloplastievorrichtung insbesondere für trikuspidale Herzklappen, wobei die Anuloplastievorrichtung mindestens zwei im oder am Anulus einer zu behandelnden Trikuspidalklappe zu implantierende oder implantierbare Verankerungselemente aufweist, welche über ein Verbindungselement miteinander verbunden sind, wobei die Länge des Verbindungselements selektiv einstellbar ist, um auf den Anulus der zu behandelnden Trikuspidalklappe eine die Form des Anulus beeinflussende Kraft auszuüben. Insbesondere ist dabei die Länge des Verbindungselements derart einstellbar, dass eine Reduktion des Anulusumfangs und einer Steigerung im Segelkoaptionsbereich bewirkbar ist.

Die erfindungsgemäße Anuloplastieprothese bzw. -Vorrichtung wird insbesondere bei Herzklappeninsuffizienzen zur Umgehung der Implantation einer künstlichen Herzklappe oder einer offenen Herzoperation eingesetzt.

Zu den anuloplastisch therapierbaren Insuffizienzen gehört vor allem der nicht stenotisch bedingte Verlust an Vorwärts-Schlagvolumen, hervorgerufen durch überhöhten systolischen Rückfluss des Blutes durch die Mitral- bzw. Trikuspidalklappe in den entsprechenden Vorhof. Für diese Insuffizienzen können chronische Ursachen wie Klappenprolaps, links- und rechtsventrikuläre Dilatation, erhöhter Druck in der Pulmonalarterie oder linksventrikuläre Klappenerkrankungen, die die Funktion der Trikuspidalklappe beeinträchtigen, vorliegen.

Für diese Insuffizienzen können akute Ursachen vorliegen wie Klappenprolaps durch Ruptur der Papillarsehnenfäden (Myxom, Endokarditis, Trauma) und Ruptur des Papillarmuskels (Infarkt, Trauma) sowie die Perforation eines Klappensegels (Endokarditis). Meistens ist aber die Trikuspidalinsuffizienz Folge einer Druckbelastung des rechten Atriums, die zu einer Anulusdeshissenz der Trikuspidalklappe führt.

Mit der erfindungsgemäßen Anuloplastievorrichtung ist eine Trikuspidalklappenrekonstruktion möglich, und zwar indem eine möglichst breite Koaptationsfläche der Klappensegel durch Verkleinerung des Trikuspidalanulus und eine Gewebeentlastung während der Systole sowie eine gute Hämodynamik während der Diastole erreicht werden. Dies beinhaltet die Korrektur der Dilatation und/oder Deformation des Anulus sowie die Prävention von wiederkehrenden Dilatationen und Deformationen.

Im Unterschied zu den bisher verwendeten, starren oder flexiblen Carpentier- bzw. Duranringen, die eine Reduzierung des Rings der Trikuspidalklappe bewirken und dadurch zu einer erhöhten Schließfähigkeit führen, sind alle Komponenten der erfindungsgemäßen Anuloplastievorrichtung hingegen minimalinvasiv am "schlagenden Herzen" implantierbar. Bei dem erfindungsgemäßen Anlass ist es - anders als bei den bekannten Carpentier- bzw. Duranringen - insbesondere nicht notwendig, die Implantation der Komponenten der Prothese an einem entlasteten und stillstehenden Anulus durchzuführen.

Dadurch, dass bei der erfindungsgemäßen Anuloplastievorrichtung mindestens zwei am Anulus der zu behandelnden Trikuspidalklappe fixierte Verankerungselement über ein Verbindungselement verbunden sind, dessen Länge selektiv einstellbar ist, weist die erfindungsgemäße Anuloplastievorrichtung den Vorteil einer zyklischen Verformbarkeit auf. Sie zeigt ferner eine hohe strukturelle Torsionsfähigkeit und eine äußerst geringe Umfangsdehnbarkeit. Dies gewährleistet eine deutliche Gewebeentlastung der atrioventrikulären Anulie.

Für die diastolische Ventrikelfüllung steht umgekehrt eine größere atrioventrikuläre Öffnungsfläche und somit ein geringerer Einströmwiderstand als bei einem völlig starren Anuloplastiering zur Verfügung.

Die folgende detaillierte Beschreibung von exemplarischen Ausführungsformen der erfindungsgemäßen Anuloplastievorrichtung ist mit Bezug auf die Zeichnungen zu lesen, in welchen gleiche Elemente in verschiedenen Zeichnungen gleich nummeriert sind. Die Zeichnungen, die nicht zwangsläufig maßstabsgetreu sind, stellen veranschaulichende Ausführungsformen dar und sind nicht dazu bestimmt, den Umfang der Erfindung zu beschränken.

Die verschiedenen hierin allgemein beschriebenen Aspekte der erfindungsgemäßen Anuloplastievorrichtung betreffen Vorrichtungen zum Behandeln von Herzstörungen oder -defekten, einschließlich beispielsweise Dilatation, Klappeninsuffizienzen sowie andere ähnliche Herzstörungen.

Die erfindungsgemäße Anuloplastievorrichtung zeichnet sich unter anderem dadurch aus, dass diese nicht nur minimalinvasiv implantierbar ist, sondern auch dadurch, dass durch Veränderung der Länge des Verbindungselements die Form und/oder Geometrie der Anuloplastievorrichtung im implantierten Zustand verändert und anwendungsabhängig an die individuelle Anatomie des zu behandelnden Herzens angepasst werden kann, um so die Effizienz des Herzens weiter zu erhöhen. Mit der erfindungsgemäßen Anuloplastievorrichtung erfährt das Herz eine gesteigerte Pumpeffizienz durch eine Alteration seiner Anulusform oder - geometrie und eine begleitende Verringerung in der Belastung der Herzwände, sowie durch eine Verbesserung der Klappenfunktion.

Obwohl die Vorrichtungen hierin in Verbindung mit deren Verwendung für die Trikuspidalklappe des Herzens diskutiert werden, können diese Vorrichtungen für andere Klappen des Herzens für ähnliche Zwecke verwendet werden, insbesondere beispielsweise für die Mitralklappe.

Die in den Zeichnungen schematisch gezeigte implantierbare Anuloplastievorrichtung kann im Allgemeinen zwei oder mehrere Verankerungselemente mit einem dort dazwischen verbundenen Verbindungselement umfassen. Die Verankerungselemente sind ausgebildet, permanent oder lösbar am Anulus der zu behandelnden Herzklappe befestigt zu werden. Die Verankerungselemente sind zueinander derart angeordnet, dass im implantierten Zustand der Anuloplastievorrichtung sich das die Verankerungselemente verbindende Verbindungselement zumindest bereichsweise über die Klappenöffnung der zu behandelnden Herzklappe spannt.

Das Verbindungselement kann selektiv gespannt oder gelöst werden, um dementsprechend die Spannung zwischen den Verankerungselementen zu beeinflussen. Demnach kann das Verbindungselement auch als "Spannelement" bezeichnet werden.

Das Verbindungselement kann eine flexible und biokompatible Multifilamentlitze in der Form beispielsweise einer Schnur oder eines Fadens umfassen. Insbesondere kann das Verbindungselement eine Kompositstruktur aufweisen, umfassend ein inneres Kabel zum Bereitstellen einer mechanischen Intaktheit und eine äußere Umhüllung zum Bereitstellen von Biokompatibilität. Das innere Kabel des Verbindungselements kann ein geflochtenes Multifilamentkabel aus Hochleistungspolymerfasern umfassen, wie beispielsweise Ultrahochmolekulargewichts-Polyethylen, verfügbar unter dem Markennamen SpectraTM und DyneemaTM, Polyester, verfügbar unter dem Markenname DacronTM oder Flüssigkristallpolymer, verfügbar unter dem Markenname VectranTM.

Die äußere Umhüllung, die das innere Kabel des Verbindungselements umgibt, kann Eigenschaften bereitstellen, die eine Dauerimplantation erleichtern, und kann folglich aus einem Material gebildet sein, das biokompatibel ist. Beispielsweise kann die äußere Umhüllung aus einem Polyestermaterial wie beispielsweise Dacron oder ePTFE hergestellt sein.

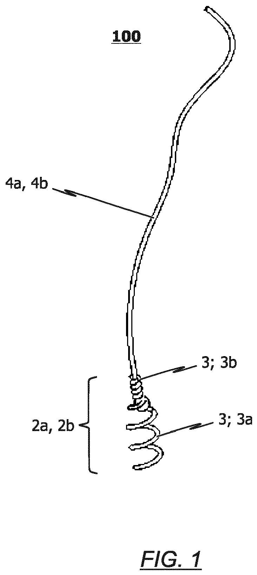

In FIG. 1 ist schematisch eine exemplarische Ausführungsform eines Verankerungselements mit einem mit dem Verankerungselement verbundenen Verbindungselement in Gestalt eines Fadens gezeigt. Das Verankerungselement ist dabei ausgebildet, eine formschlüssige Verbindung mit dem Anulus einer zu behandelnden Herzklappe, insbesondere Trikuspidalklappe auszubilden. Zu diesem Zweck weist das Verankerungselement einen schraubenspindelartigen Bereich auf, welcher minimalinvasiv in das Gewebe am Anulus der zu behandelnden Herzklappe einbringbar ist.

Der Darstellung in FIG. 1 ist insbesondere zu entnehmen, dass der schraubenspindelartige Bereich als Wendel oder Schraube ausgeführt sein kann. Insbesondere ist der schraubenspindelartige Bereich in Form eines gebogenen Drahtes ausgeführt. Der Drahtdurchmesser beträgt vorzugsweise etwa 0,2 bis 0,7 mm und noch bevorzugter etwa 0,4 bis 0,6 mm.

Allgemein kommt dem Verankerungselement die Funktion des Befestigens des Verbindungselements am Anulus des zu behandelnden Herzens zu.

Bei der in FIG. 1 schematisch dargestellten Ausführungsform weist das Verankerungselement einen ersten Bereich mit einem ersten effektiven Durchmesser und einen zweiten Bereich mit einem im Vergleich zum ersten Durchmesser verringerten Durchmesser aufweist. Bei dem in FIG. 1 gezeigten Ausführungsbeispiel, bei welchem der schraubenspindelartige Bereich in Form eines gebogenen Drahtes ausgeführt ist, variiert beispielsweise der Wicklungsdurchmesser von etwa 2 bis 4 mm auf etwa 1 bis 0,5 mm, und die Steigung variiert von ca. 1,5 mm auf ca. 0.5 mm.

Der Darstellung in FIG. 1 ist ferner zu entnehmen, dass das als Faden ausgeführte Verbindungselement über einen Knoten mit dem Verankerungselement verbunden ist, wobei der Knoten in der engen Wicklung des schraubenspindelartigen Bereiches liegt, so dass die enge Wicklung und der Knoten das Verbindungselement halten. Der Haltepunkt am Verankerungselement ist asymmetrisch angebracht, so dass unter Zug des Verbindungselements das Verankerungselement nicht herausgedreht werden kann.

Zum minimalinvasiven Implantieren des Verankerungselements mit dem daran befestigten Verbindungselement kommt ein Einführmechanismus mit einem kathetergeführten Werkzeug zum Einsatz, welches ausgebildet ist, eine formschlüssige Verbindung zwischen dem Verankerungselement und dem Gewebe am Anulus der zu behandelnden Herzklappe auszubilden.

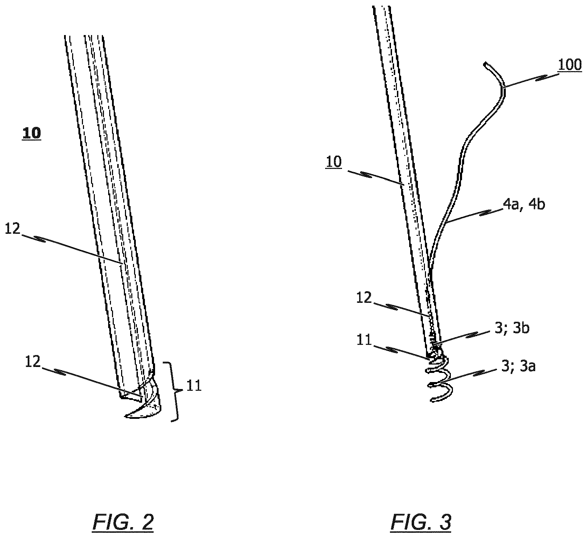

Gemäß dem in FIG. 2 teilweise gezeigten Ausführungsbeispiel des kathetergeführten Werkzeuges weist dieses am distalen Endbereich einen hohlkörperförmigen Bereich mit einer Wendelnut auf. Die Wendelnut ist dabei derart an das am Anulus der zu behandelnden Herzklappe zu verankernde Verankerungselement angepasst ist, dass durch eine Rotation des hohlkörperförmigen Bereiches um seine Längsachse das Verankerungselement zumindest bereichsweise in das Gewebe am Anulus einführbar ist.

FIG. 3 zeigt schematisch die exemplarische Ausführungsform des kathetergeführten Werkzeuges gemäß FIG. 2 mit einem im distalen Endbereich des Werkzeuges zumindest bereichsweise aufgenommenen Verankerungselement. Demnach wird bei dieser Ausführungsform das kathetergeführte Werkzeug auf den Teilbereich des schraubenspindelartigen Bereiches des Verankerungselements gedreht, an dem sich der Durchmesser des schraubenspindelartigen Bereiches ändert.

Ebenfalls ist der Darstellung in FIG. 3 zu entnehmen, dass der hohlkörperförmige Bereich des kathetergeführten Werkzeuges eine seitliche Schlitzöffnung aufweist zum zumindest teilweisen Aufnehmen des Verbindungselements.

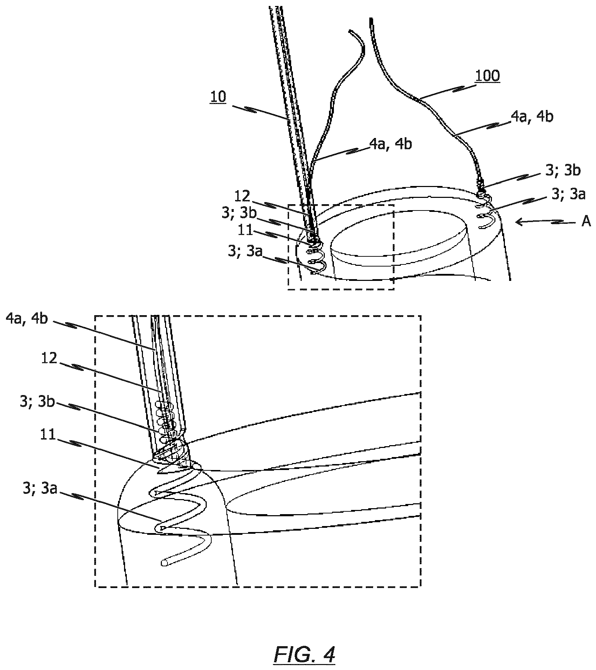

In FIG. 4 ist schematisch der Vorgang zum Implantieren der Verankerungselemente mit Hilfe des kathetergeführten Werkzeuges gemäß FIG. 2 am Anulus einer zu behandelnden Herzklappe gezeigt. Im Einzelnen wird zur Implantation der erfindungsgemäßen Anuloplastievorrichtung jeweils mindestens ein Verankerungselement an mindestens zwei Positionen des Anulus der zu behandelnden Herzklappe in das Gewebe des Anulus eingedreht.

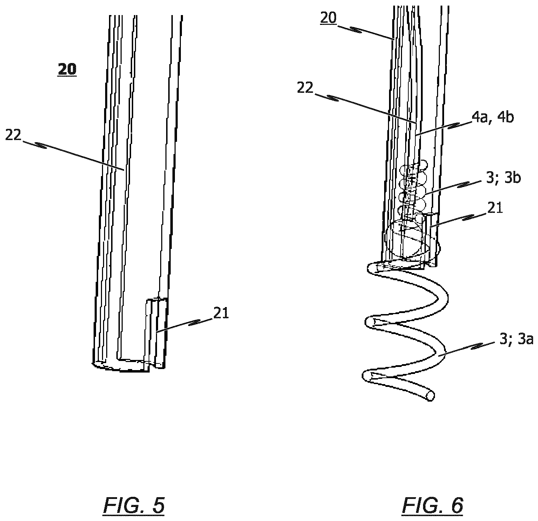

FIG. 5 zeigt bereichsweise eine weitere exemplarische Ausführungsform des distalen Endbereiches eines kathetergeführten Werkzeuges zum Ausbilden einer formschlüssigen Verbindung zwischen einem Verankerungselement und dem Gewebe am Anulus einer zu behandelnden Trikuspidalklappe. Gemäß dieser Ausführungsform weist das kathetergeführte Werkzeug einen hohlkörperförmigen Bereich mit einer Längsnut auf, wobei die Längsnut derart an das am Anulus der zu behandelnden Trikuspidalklappe verankernde Verankerungselement angepasst ist, dass durch eine Rotation des hohlkörperförmigen Bereiches um seine Längsachse das Verankerungselement zumindest bereichsweise in das Gewebe am Anulus der zu behandelnden Trikuspidalklappe einführbar ist.

Der Darstellung in FIG. 5 ist ferner zu entnehmen, dass der distale Endbereich des kathetergeführten Werkzeuges optional einen seitlichen Schlitz (seitliche Schlitzöffnung) aufweist zum Einlegen des fadenartigen Verbindungselements. Ohne solch eine seitliche Schlitzöffnung müsste das Verbindungselement entsprechend durch das Werkzeug gefädelt werden.

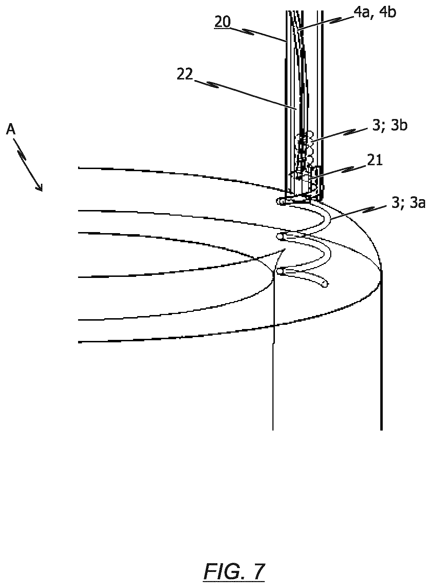

In FIG. 6 ist schematisch die exemplarische Ausführungsform des kathetergeführten Werkzeuges gemäß FIG. 5 mit einem im distalen Endbereich des Werkzeuges zumindest bereichsweise aufgenommenen Verankerungselement gezeigt, während FIG. 7 schematisch den Vorgang zum Implantieren der Verankerungselemente mit Hilfe des kathetergeführten Werkzeuges gemäß FIG. 5 am Anulus einer zu behandelnden Herzklappe darstellt.

Demnach ist ersichtlich, dass - im Unterschied zu der exemplarischen Ausführungsform gemäß FIG. 2 - bei der Ausführungsform des kathetergeführten Werkzeuges gemäß FIG. 5 das Werkzeug auf den Teilbereich des schraubenspindelartigen Bereiches des Verankerungselements, an dem sich der Durchmesser ändert, gesteckt wird. Auf diese Weise dreht das Werkzeug in zwei Richtungen und ermöglicht damit das Rein- und Rausdrehen des schraubenspindelartigen Bereiches des Verankerungselements in das bzw. aus dem Gewebe am Anulus der zu behandelnden Herzklappe.

Zum Rausdrehen des schraubenspindelartigen Bereiches des Verankerungselements aus dem Gewebe am Anulus der zu behandelnden Herzklappe wird das nachträgliche Aufsetzen des Werkzeuges dadurch erleichtert, dass das Werkzeug seitlich keinen Schlitz hat, so dass das am Verankerungselement befestigte Verbindungselement (Faden) als Führung für das Werkzeug verwendet werden kann.

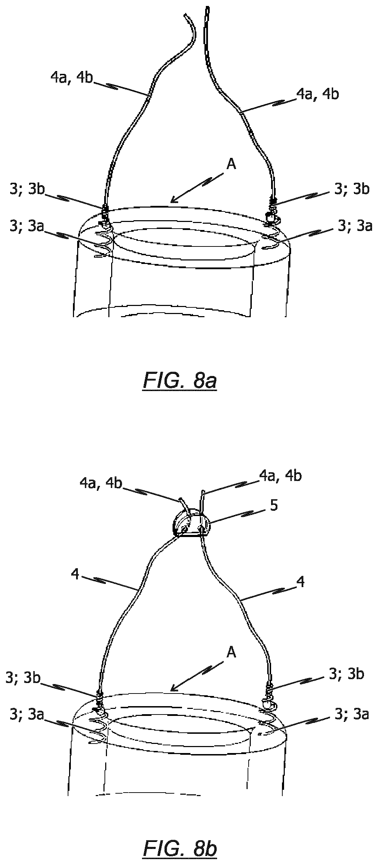

FIG. 8a zeigt schematisch einen Zustand, in welchem bereits zwei Verankerungselemente, an denen jeweils ein Verbindungselement in Gestalt eines Fadens befestigt ist, am Anulus der zu behandelnden Herzklappe befestigt sind. In diesem Zustand kann die Zugfestigkeit der Verbindung von außen geprüft werden.

FIG. 8b bis FIG. 8g zeigen schematisch den Vorgang zum Verbinden der beiden am Anulus der zu behandelnden Herzklappe implantierten Verankerungselementen, an denen jeweils ein Verbindungselement in Gestalt eines Fadens befestigt ist. Hierzu kommt ein Befestigungselement zum Einsatz, welches dazu dient, die beiden Verbindungselemente der implantierten Verankerungselemente miteinander zu verbinden.

Wie in FIG. 8b gezeigt, kann das Befestigungselement als eine Art Befestigungslasche ausgebildet sein, welche auf die in Gestalt von Fäden ausgeführten Verbindungselemente aufschiebbar ist.

Bei der in FIG. 8b gezeigten Ausführungsform hat das Befestigungselement (Befestigungslasche) einen Durchmesser von etwa 1 bis 3 mm und weist zwei Bohrungen jeweils mit einem größeren Durchmesser als der Durchmesser der Verbindungselemente (Fäden) auf. Diese Bohrungen halten und führen die Verbindungselemente (Fäden).

Anstelle einer Befestigungslasche sind als Befestigungselement aber auch andere Ausführungsformen denkbar, wie etwa Krimp-Elemente oder Knoten. Allgemein ausgedrückt sollte das Befestigungselement ausgebildet sein, die beiden Verbindungselemente derart zu verbinden, dass die Länge des die beiden Verankerungselemente letztendlich verbindenden Verbindungselements selektiv einstellbar ist.

Die Länge des die beiden Verankerungselemente letztendlich verbindenden Verbindungselements sollte vorzugsweise derart einstellbar sein, dass im implantierten Zustand der Anuloplastievorrichtung eine auf den Anulus der zu behandelnden Herzklappe einwirkende Kraft erzeugt wird, wodurch die Klappenfunktion der zu behandelnden Herzklappe verändert wird.

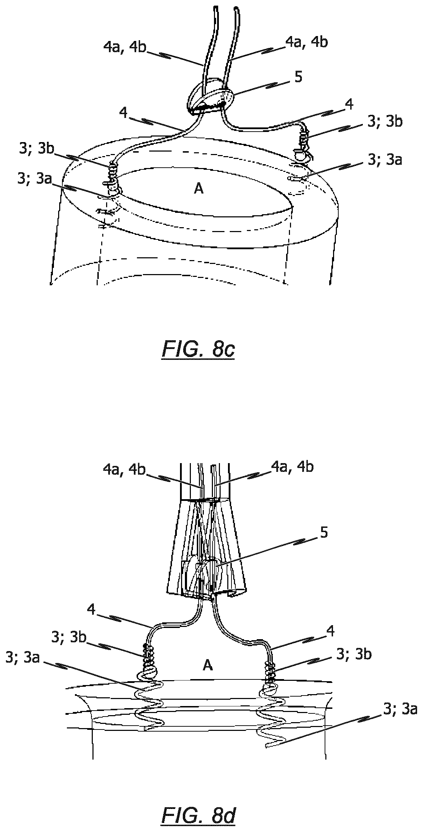

Dieser Vorgang ist schematisch in FIG. 8c und FIG. 8d gezeigt. Im Einzelnen ist in FIG. 8c angedeutet, wie die Länge des die beiden Verankerungselemente letztendlich verbindenden Verbindungselements im Hinblick auf die zu behandelnde Herzklappe abgestimmt/justiert wird. Demnach werden durch ein Bewegen des Befestigungselements in proximaler oder distaler Richtung dessen Position und die Länge des die beiden Verankerungselemente letztendlich verbindenden Verbindungselements, sowie die auf den Anulus der Herzklappe wirkende mechanische "Vorspannung" mit Hilfe eines Greifwerkzeuges eingestellt.

Im Einzelnen wird mit Hilfe des Greifwerkzeuges die auf den Anulus der Herzklappe wirkende mechanische "Vorspannung" auf optimalen Druckaufbau im Herzen justiert.

Anschließend wird der Justierzustand des Befestigungselements fixiert.

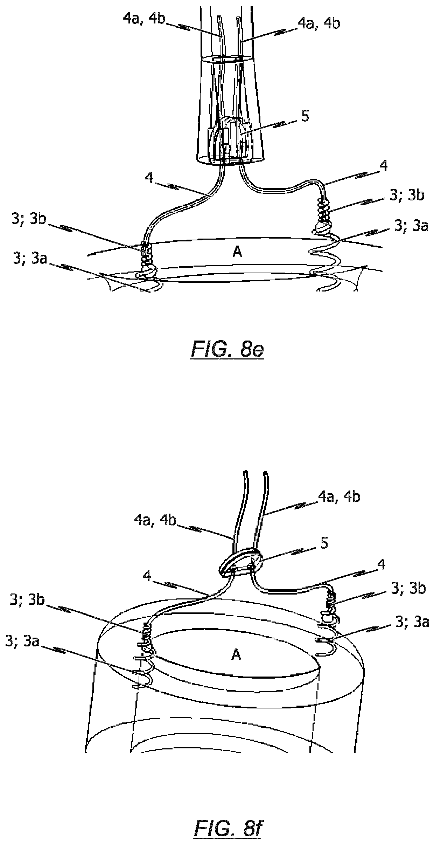

Wie in FIG. 8e angedeutet, kann dies beispielsweise durch Krimpen (Zusammenquetschen) des Befestigungselements erfolgen. Das Zusammenquetschen erfolgt vorzugsweise mit dem Greifwerkzeug, welches wie eine Biopsiezange ausgeführt sein kann. Vorzugsweise hat das Greifwerkzeug eine axiale Bohrung, über welche das Greifwerkzeug auf die jeweils als Faden ausgebildeten Verbindungselemente der Verankerungselemente geführt wird.

In FIG. 8f ist das gequetschte Befestigungselement (Krimphülse) dargestellt.

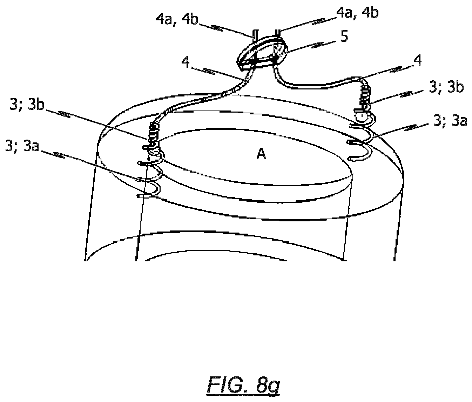

Anschließend werden - wie in FIG. 8g angedeutet - die überflüssigen Fäden mit einem handelsüblichen Abschneid-Werkzeug abgeschnitten.

Da die Verbindungselemente jeweils an dem Teilbereich des schraubenspindelartigen Bereiches des Verankerungselements befestigt sind, welcher den kleineren Durchmesser aufweist, sind diese federnd mit dem jeweiligen Verankerungselement verbunden. Insbesondere wirkt der Teilbereich des schraubenspindelartigen Bereiches des Verankerungselements, welcher den kleineren Durchmesser aufweist, als Feder, so dass die Fadenspannung nur langsam aufgebaut wird. Über die Länge des Teilbereiches des schraubenspindelartigen Bereiches mit dem kleineren Durchmesser kann die Geschwindigkeit des Anstieges der Fadenspannung eingestellt werden. Sind die im implantierten Zustand der Verankerungselemente freien Federenden kürzer ausgeführt, wird die Fadenspannung schneller ansteigen.

Die außeraxiale Belastung der Verankerungselemente und die exzentrische Wicklung der schraubenspindelartigen Bereiche der Verankerungselemente verhindern ein ungewolltes Herausdrehen der Verankerungselemente.

Die Erfindung ist nicht auf die in den Zeichnungen schematisch dargestellten Ausführungsformen beschränkt, sondern ergibt sich aus einer Zusammenschau sämtlicher hierin offenbarter Merkmale und Einzelaspekte, die beliebig miteinander kombiniert werden können.

## Patentansprüche

1. Anuloplastievorrichtung (100) zur minimalinvasiven Behandlung einer dysfunktionellen Herzklappe, insbesondere Trikuspidalklappe eines Patienten, wobei die Anuloplastievorrichtung (100) Folgendes aufweist:
- ein erstes Verankerungselement (2a), welches ausgebildet ist, eine formschlüssige Verbindung mit dem Gewebe am Anulus (A) der zu behandelnden Herzklappe auszubilden;
- mindestens ein zweites Verankerungselement (2b), welches ausgebildet ist, eine Verbindung mit dem Anulus (A) der zu behandelnden Herzklappe auszubilden; und
- ein Verbindungselement (4; 4a, 4b), welches das erste Verankerungselement (2a) mit dem mindestens einen zweiten Verankerungselement (2b) verbindet,
wobei die Länge des Verbindungselements (4; 4a, 4b) selektiv einstellbar ist.

2. Anuloplastievorrichtung (100) nach Anspruch 1,
wobei das erste und das mindestens eine zweite Verankerungselement (2a, 2b) jeweils einen schraubenspindelartigen Bereich (3) aufweisen, welcher minimalinvasiv in das Gewebe am Anulus (A) der zu behandelnden Herzklappe einbringbar ist.

3. Anuloplastievorrichtung (100) nach Anspruch 2,
wobei der schraubenspindelartige Bereich (3) als Wendel oder Schraube ausgeführt ist.

4. Anuloplastievorrichtung (100) nach einem der Ansprüche 1 bis 3,
wobei die Verankerungselemente (2a, 2b) jeweils einen ersten Bereich (3a) mit einem ersten effektiven Durchmesser und einen zweiten Bereich (3b) mit einem im Vergleich zum ersten Durchmesser verringerten Durchmesser aufweisen.

5. Anuloplastievorrichtung (100) nach einem der Ansprüche 1 bis 4,
wobei das Verbindungselement (4; 4a, 4b) an einem Verbindungspunkt mit dem ersten Verankerungselement (2a) fest verbunden ist, wobei der Verbindungspunkt einer bezüglich des ersten Verankerungselements (2a) asymmetrischen Position liegt.

6. Anuloplastievorrichtung (100) nach einem der Ansprüche 1 bis 5,
wobei das Verbindungselement (4; 4a, 4b) mehrteilig ausgeführt ist und einen ersten Bereich (4a) aufweist, welcher mit dem ersten Verankerungselement (2a) fest verbunden ist, und mindestens einen weiteren, zweiten Bereich (4b) aufweist, welcher mit dem mindestens einen zweiten Verankerungselement (2b) fest verbunden ist, wobei die Anuloplastievorrichtung (100) ferner ein Befestigungselement (5) aufweist zum Verbinden des ersten Bereiches (4a) des Verbindungselements mit dem mindestens einen zweiten Bereich (4b) des Verbindungselements.

7. Anuloplastievorrichtung (100) nach Anspruch 6,
wobei das Befestigungselement (5) ausgebildet ist, den ersten Bereich (4a) des Verbindungselements mit dem mindestens einen zweiten Bereich (4b) des Verbindungselements derart zu verbinden, dass letztendlich die Länge des Verbindungselements (4) selektiv einstellbar ist, wobei die Länge des Verbindungselements (4) vorzugsweise derart einstellbar ist, dass im implantierten Zustand der Anuloplastievorrichtung (100) eine auf den Anulus (A) der zu behandelnden Herzklappe des Patienten einwirkende Kraft erzeugt wird, wodurch die Klappenfunktion der zu behandelnden Herzklappe verändert wird.

8. Anuloplastievorrichtung (100) nach Anspruch 6 oder 7,
wobei das Befestigungselement (5) ausgebildet ist, den ersten Bereich (4a) des Verbindungselements mit dem mindestens einen zweiten Bereich (4b) des Verbindungselements kraftschlüssig, insbesondere klemmend, zu verbinden.

9. Anuloplastievorrichtung (100) nach einem der Ansprüche 1 bis 8,
wobei das Verbindungselement (4; 4a, 4b) biegsam in Längsrichtung jedoch nicht oder zumindest im Wesentlichen nicht elastisch ausgeführt ist und insbesondere in Gestalt eines mehrere miteinander verbundene oder verdrehte Fasern aufweisenden Fadens ausgeführt ist.

10. System zur minimalinvasiven Behandlung einer dysfunktionellen Herzklappe eines Patienten, wobei das System Folgendes aufweist:
- eine Anuloplastievorrichtung (100) nach einem der Ansprüche 1 bis 9; und
- einen Einführmechanismus zum minimalinvasiven Einführen und Implantieren der einzelnen Komponenten der Anuloplastievorrichtung (100) in dem zu behandelnden Herzen des Patienten.

11. System nach Anspruch 10,
wobei der Einführmechanismus ein kathetergeführtes Werkzeug (10, 20) aufweist zum Ausbilden der formschlüssigen Verbindung zwischen dem ersten und/oder dem mindestens einen zweiten Verankerungselement (2a, 2b) und dem Gewebe am Anulus (A) der zu behandelnden Herzklappe.

12. System nach Anspruch 11,
wobei das kathetergeführte Werkzeug (10) einen hohlkörperförmigen Bereich mit einer Wendelnut (11) aufweist, wobei die Wendelnut (11) derart an das im Gewebe am Anulus (A) der zu behandelnden Herzklappe zu verankernde Verankerungselement (2a, 2b) angepasst ist, dass durch eine Rotation des hohlkörperförmigen Bereiches um seine Längsachse das Verankerungselement (2a, 2b) zumindest bereichsweise in das Gewebe am Anulus (A) der zu behandelnden Herzklappe einführbar ist.

13. System nach Anspruch 11,
wobei das kathetergeführte Werkzeug (20) einen hohlkörperförmigen Bereich mit einer Längsnut (21) aufweist, wobei die Längsnut (21) derart an das im Gewebe am Anulus (A) der zu behandelnden Herzklappe zu verankernde Verankerungselement (2a, 2b) angepasst ist, dass durch eine Rotation des hohlkörperförmigen Bereiches um seine Längsachse das Verankerungselement (2a, 2b) zumindest bereichsweise in das Gewebe am Anulus (A) der zu behandelnden Herzklappe einführbar ist.

14. System nach Anspruch 12 oder 13,
wobei der hohlkörperförmige Bereich des kathetergeführten Werkzeuges (10, 20) eine seitliche Schlitzöffnung (12, 22) aufweist zum zumindest teilweisen Aufnehmen des Verbindungselements (4; 4a, 4b).

15. System nach einem der Ansprüche 12 bis 14,
wobei der hohlkörperförmige Bereich des kathetergeführten Werkzeuges (10, 20) ausgebildet ist, das erste oder zweite Verankerungselement (2a, 2b) derart bereichsweise aufzunehmen, dass ein Endbereich des Verankerungselements (2a, 2b) stirnseitig aus dem hohlkörperförmigen Bereich heraussteht.
